(19) Europäisches Patentamt
European Patent Office
Office européen des brevets

(11) **EP 3 722 783 A1**

(12) **EUROPEAN PATENT APPLICATION**
published in accordance with Art. 153(4) EPC

(43) Date of publication:
**14.10.2020 Bulletin 2020/42**

(21) Application number: **18886452.4**

(22) Date of filing: **30.11.2018**

(51) Int Cl.:
*G01N 15/06* (2006.01)

(86) International application number:
**PCT/CN2018/118699**

(87) International publication number:
**WO 2019/109874 (13.06.2019 Gazette 2019/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.12.2017 CN 201711269390**

(71) Applicant: **Fatri United Testing & Control (Quanzhou) Technologies Co., Ltd.**
**Fujian 362000 (CN)**

(72) Inventors:
• **NIE, Yongzhong**
  **Fujian 361008 (CN)**
• **ZHANG, Zhongping**
  **Fujian 361008 (CN)**

(74) Representative: **Angerhausen, Christoph**
**Boehmert & Boehmert**
**Anwaltspartnerschaft mbB**
**Pettenkoferstrasse 22**
**80336 München (DE)**

(54) **DETECTION METHOD FOR CONCENTRATION OF FLUID PARTICULATE MATTER**

(57)    The present invention discloses a method for detecting a particle concentration in a fluid, including: S1: introducing a pure fluid into a detection device, and obtaining a scatter background noise value $U_{background-noise}$ outputted by the detection device; S2: introducing a fluid under test into the detection device, obtaining a scattered signal outputted by the detection device and obtaining a voltage signal of a standard particle; S3: performing signal sampling on the fluid for a period of time, extracting an valid signal Ux, and performing a threshold analysis on the sampled valid signal to obtain a number of particles appearing during the period of time; S4: obtaining a particle concentration in the fluid according to the number of particles obtained in S3.

**Description**

TECHNICAL FIELD

The invention relates to the technical field of detection device, in particular to a method for detecting a particle concentration in a fluid.

BACKGROUND

In the operation process of an engine, a bearing, a gear and so on, a lubricating oil system is often required to be arranged so as to reduce abrasion loss, and factors such as insufficient purity of lubricating oil, existence of debris in lubricating oil will cause great disaster. A failed operation process of a large-scale mechanical equipment is often due to a vicious cycle caused by a continuous accumulation of abrasive debris in the lubricating oil. According to a diagnosis system for abrasion fault, there is a strong correlation between a damage degree of a worn component (for example, an engine, a rolling bearing, a gear, etc.) and particles in the lubricating system. In order to realize the detection of particles in the lubricating oil, a particle form detection apparatus, such as an apparatus for detecting particle concentration in a fluid, is usually arranged in a lubricating oil pipeline to monitor the quality of the lubricating oil on-line in real time, so as to provide an effective basis for fault diagnosis of an engine, a bearing, a gear, etc., and quickly and accurately determine the abrasion state and the reason of the fault.

However, in existing methods for detecting particle concentration in a fluid, elimination of an influence of a background noise value of the fluid itself is lacked, which leads to a large error in subsequent calculation of particle concentration, thereby affecting the accuracy of the calculation of the concentration; additionally, there are defects in the selection of a standard particle, so that measurement accuracy and sensitivity cannot be pursued at the same time in the measurement process; finally, in the calculation of the concentration, there is no correction of the influence of background noise value, which further affects the measurement accuracy.

SUMMURY

In order to overcome the disadvantages of the prior art, the technical problem solved by the present invention is: how to provide a detection method for more accurately measuring particle concentration of in a fluid.

In order to solve the above technical problem, the technical solutions adopted by the present invention are as follows:

> S1: introducing a pure fluid into a detection device, and obtaining a scatter background noise value $U_{background-noise}$ outputted by the detection device;
> S2: introducing a fluid under test into the detection device, obtaining a scattered signal outputted by the detection device and obtaining a voltage signal of a standard particle;
> S3: performing signal sampling on the fluid for a period of time, extracting an valid signal $U_X$ from sampled signals, and performing a threshold analysis on the valid signal $U_X$ obtained by sampling to obtain a number of particles appearing during the period of time;
> S4: obtaining a particle concentration in the fluid according to the number of particles obtained in S3.

It should be noted that, in order to solve the defects in the prior art mentioned in the above background section, the inventor has made several improvements to the detection method of the particle concentration in the fluid, including: obtaining the scattering background noise value $U_{background-noise}$ outputted by the detection device, and eliminating the influence caused by the background noise value in the subsequent detection and calculation process, so as to improve the accuracy of the detection and calculation of the particle concentration in the fluid.

It should be noted that, the period of time may refer to any time period and may be selected according to an actual situation.

Preferably, a particle with a diameter of 10 $\mu$m is selected as the standard particle, and the voltage signal corresponding to the standard particle is $U_{10\mu m}$.

According to the technical solution, the particle with a diameter of 10 $\mu$m is preferably selected as the standard particles, which can improve the detection accuracy on the one hand and improve the detection sensitivity on the other hand. When the particle size is too large, the accuracy of the detection of subsequent concentration calculation will be reduced, and when the particle size is too small, the detection sensitivity of the device will be reduced, resulting in the situation where the particle size cannot be detected. Therefore, the inventor uses the particle with a diameter of 10 $\mu$m as the standard particle, so that the detection accuracy and detection sensitivity can be effectively balanced, making the detection process more accurate.

Preferably, the extracting the valid signal includes: comparing sampled signals with the scatter background noise value, and selecting a signal greater than the scatter background noise value as the valid signal.

According to the technical solution, it is necessary to select the valid signal as the basis for subsequent calculation, otherwise the accuracy of the result of the detection and calculation will be affected. The inventor adopts a simple and effective manner for selecting the valid signal, that is, the sampled signals are compared with the previously obtained scattering background noise value, and the signal greater than the scatter background noise value is selected as the valid signal, so that the sampled signals are more useful in practice and the results of subsequent measurement are more accurate.

Preferably, the performing the threshold analysis to obtain the number of particles in S3 includes:

comparing the sampled signals Ux with the scatter background noise value $U_{background-noise}$;
incrementing a count of the number of particles by 1 when $U_X - U_{background-noise} > 0$; and
incrementing the count of the number of particles by 0, when $U_X - U_{background-noise} < 0$.

In this step, preferably, the inventor chooses to count by comparing the signal value with the background noise value rather than counting based on the numbers that are read according to the signal value, so as to eliminate the error caused by the background noise value, that is, only when $U_X - U_{background-noise} > 0$, the signal would be counted as a particle, which makes the detection result more accurate and improves the accuracy of the detection of the particle concentration.

Preferably, the obtaining the particle concentration in S4 includes:

S41: calculating a volume Vx of a particle:

$$V_X = K * V_{10um} * \sqrt{\frac{U_X - U_{background-noise}}{U_{10um} - U_{background-noise}}}$$

where, $V_X$ is the volume of the unknown particle; K is a sensor correction coefficient; $V_{10um}$ is a volume of the standard particle; $U_X$ is an output voltage amplitude of the unknown particle; $U_{10um}$ is an output voltage amplitude of the standard particle; and
S42: obtaining the particle concentration in the fluid:

obtaining a flow velocity v of the fluid, and a cross-sectional area S of a detection pipeline, converting the number of the particles passing through during the period of time t and the volumes of the particles into a total mass m, and obtaining the particle concentration c by the following formula:

$$c = \frac{M}{v * t * S} (ug/m^3)$$

It should be noted that, in the calibration process of the sensor, inevitably, calibration deviation of the background noise occurs and the measurement error occurs, and the sensor correction coefficient K refers to a fine-tuning correction coefficient K that is introduced for fine-tuning in the above situation; it is also possible that when the standard particle is selected, the particle is not fully standard, resulting in some small volume calculation errors, which can also be corrected if the correction coefficient is introduced.

In this step, the consideration of removing the influence of background noise value is also included, which makes the detection result more accurate. For example, the above calculation formula includes subtracting $U_{background-noise}$ from $U_X$ and subtracting $U_{background-noise}$ from $U_{10um}$, which can make the calculated particle volume closer to the actual value and improve the accuracy of the calculation of the particle concentration in the fluid.

Compared with the prior art, the invention has the advantages of:

1. In the method for detecting a particle concentration in a fluid according to the present invention, in the calculation step of the particle concentration, the consideration of removing the influence of the background noise value is included, which can make the calculated particle volume closer to the actual value and improve the accuracy of the calculation of the particle concentration in the fluid;
2. In the method for detecting a particle concentration in a fluid according to the present invention, the particle with a diameter of 10 μm is selected as the standard particle, which can improve the detection accuracy on the one hand and improve the detection sensitivity on the other hand;

3. In the method for detecting a particle concentration in a fluid according to the present invention, the sampled signals are compared with the previously obtained scattering background noise value, and the signal greater than the scatter background noise value is selected as the valid signal, so that the sampled signals are more useful in practice and the results of subsequent measurement are more accurate.

4. In the method for detecting a particle concentration in a fluid according to the present invention, the inventor chooses to count by comparing the signal value with the background noise value rather than counting based on the numbers that are read according to the signal value, which makes the detection result more accurate and improves the accuracy of the detection of the particle concentration;

The foregoing description is only an overview of the technical solutions of the present invention, and in order to make the technical means of the present invention more clearly understood and the present invention can be implemented in accordance with the content of the description, and in order to make the above and other objects, features, and advantages of the present invention more clearly understood, the following preferred embodiments are specifically described below with reference to the accompanying drawings.

DESCRIPTION OF EXAMPLE CONFIGURATIONS

To further illustrate the technical means and effects of the present invention adopted to achieve the predetermined objects, embodiments, structures, characteristics and effects according to the present invention are described with reference to the accompanying drawings and preferred embodiments:

The present invention relates to a method for detecting a particle concentration in a fluid, which includes the following steps:

S1: introducing a pure fluid into a detection device, and obtaining a scatter background noise value $U_{background-noise}$ outputted by the detection device;

S2: introducing a fluid under test into the detection device, obtaining a scattered signal outputted by the detection device and obtaining a voltage signal of a standard particle;

S3: performing signal sampling on the fluid for a period of time, extracting an valid signal $U_X$ from sampled signals, and performing a threshold analysis on the valid signal $U_X$ obtained by sampling to obtain a number of particles appearing during the period of time;

S4: obtaining a particle concentration in the fluid according to the number of particles obtained in S3.

The scattering background noise value $U_{background-noise}$ outputted by the detection device is obtained, and the influence caused by the background noise value is eliminated in the subsequent detection and calculation process, which improves the accuracy of the detection and calculation of the particle concentration in the fluid.

In combination with the above implementation, in one of the preferred embodiments, a particle with a diameter of 10 $\mu$m is selected as the standard particle, and the voltage signal corresponding to the standard particle is $U_{10\mu m}$.

In the actual process of selecting the standard particle, when the particle size is too large, the accuracy of the detection of subsequent concentration calculation will be reduced, and when the particle size is too small, the sensitivity of the detection by the device will be reduced, resulting in the situation where the particle size cannot be detected. Therefore, the inventor uses the particle with a diameter of 10 $\mu$m as the standard particle, so that the detection accuracy and detection sensitivity can be effectively balanced, making the detection process more accurate. On the one hand, the detection accuracy can be improved, on the other hand, the detection sensitivity can be improved.

In combination with the above embodiments, in one of the preferred embodiments, the extracting the valid signal includes: comparing sampled signals with the scatter background noise value, and selecting a signal greater than the scatter background noise value as the valid signal.

The sampled signals are compared with the previously obtained scattering background noise value, and the signal greater than the scatter background noise value is selected as the valid signal, so that the sampled signals are more useful in practice and the results of subsequent measurement are more accurate.

In combination with the above embodiments, in one of the preferred embodiments, the performing the threshold analysis to obtain the number of particles in S3 includes:

comparing the sampled signals Ux with the scatter background noise value $U_{background-noise}$; incrementing a count of the number of particles by 1 when $U_X - U_{background-noise} > 0$; and incrementing the count of the number of particles by 0, when $U_X - U_{background-noise} < 0$.

In this step, preferably, the inventor chooses to count by comparing the signal value with the background noise value rather than counting based on the numbers that are read according to the signal value, so as to eliminate the error caused by the background noise value, that is, only when $U_X - U_{background-noise} > 0$, the signal would be counted as a particle, which makes the detection result more accurate and improves the accuracy of the detection of the particle concentration.

In combination with the above embodiments, in another preferred embodiment, the obtaining the particle concentration in S4 includes:

S41: calculating a volume Vx of a particle:

$$V_X = K * V_{10um} * \sqrt{\frac{U_X - U_{background-noise}}{U_{10um} - U_{background-noise}}}$$

where, $V_X$ is the volume of the unknown particle; K is a sensor correction coefficient; $V_{10um}$ is a volume of the standard particle; $U_X$ is an output voltage amplitude of the unknown particle; $U_{10um}$ is an output voltage amplitude of the standard particle; and

S42: obtaining the particle concentration in the fluid:

obtaining a flow velocity v of the fluid, and a cross-sectional area S of a detection pipeline, converting the number of the particles passing through during the period of time t and the volumes of the particles into a total mass m, and obtaining the particle concentration c by the following formula:

$$c = \frac{M}{v * t * S} (ug/m^3)$$

In this step, the consideration of removing the influence of background noise value is also included, which makes the detection result more accurate. For example, the above calculation formula includes subtracting $U_{background-noise}$ from $U_X$ and subtracting $U_{background-noise}$ from $U_{10\mu m}$, which can make the calculated particle volume closer to the actual value and improve the accuracy of the calculation of the particle concentration in the fluid.

The total mass m is calculated as follows:

Calculating an individual particle:

$$\mathrm{m} = \rho * V$$

Here, the particle is a common particle in the fluid by default, and the mass of the individual particle can be obtained by substituting the relative density thereof.

Based on the calculation of the individual particle, the total mass of the particles passing through in a period of time can be obtained by accumulating the mass of the particles passing through in the period of time:

$$c = \frac{M}{v * t * S} (ug/m^3)$$

The above embodiments are only the preferred embodiments of the present invention, and cannot be used to define the protection scope of the invention. Any non-substantive changes and replacements made by those skilled in the art on the basis of the present invention belong to the protection scope claimed by the present invention.

**Claims**

1. A method for detecting a particle concentration in a fluid, comprising:

S1: introducing a pure fluid into a detection device, and obtaining a scatter background noise value $U_{background-noise}$ outputted by the detection device;

S2: introducing a fluid under test into the detection device, obtaining a scattered signal outputted by the detection device and obtaining a voltage signal of a standard particle;

S3: performing signal sampling on the fluid for a period of time, extracting an valid signal $U_X$ from sampled signals, and performing a threshold analysis on the valid signal $U_X$ obtained by sampling to obtain a number of particles appearing during the period of time;

S4: obtaining a particle concentration in the fluid according to the number of particles obtained in S3.

2. The method according to claim 1, wherein, a particle with a diameter of 10 μm is selected as the standard particle, and the voltage signal corresponding to the standard particle is U10μm.

3. The method according to claim 2 or claim 3, wherein, the extracting the valid signal comprises: comparing sampled signals with the scatter background noise value, and selecting a signal greater than the scatter background noise value as the valid signal.

4. The method according to any one of claims 1 to 3, wherein, the performing the threshold analysis to obtain the number of particles in S3 comprises:

comparing the sampled signals $U_X$ with the scatter background noise value $U_{background\text{-}noise}$,
incrementing a count of the number of particles by 1 when $U_X - U_{background\text{-}noise} > 0$; and
incrementing the count of the number of particles by 0, when $U_X - U_{background\text{-}noise} < 0$.

5. The method according to any one of claims 1 to 4, wherein, the obtaining the particle concentration in S4 comprises:

S41: calculating a volume Vx of a particle:

$$V_X = K * V_{10um} * \sqrt{\frac{U_X - U_{background-noise}}{U_{10um} - U_{background-noise}}}$$

wherein, $V_X$ is the volume of the unknown particle; K is a sensor correction coefficient; $V_{10um}$ is a volume of the standard particle; $U_X$ is an output voltage amplitude of the unknown particle; $U_{10um}$ is an output voltage amplitude of the standard particle; and
S42: obtaining the particle concentration in the fluid:

obtaining a flow velocity v of the fluid, and a cross-sectional area S of a detection pipeline, converting the number of the particles passing through during the period of time t and the volumes of the particles into a total mass m, and obtaining the particle concentration c by the following formula:

$$c = \frac{M}{v * t * S} (ug/m^3)$$

## INTERNATIONAL SEARCH REPORT

| | International application No. |
|---|---|
| | **PCT/CN2018/118699** |

**A.    CLASSIFICATION OF SUBJECT MATTER**

G01N 15/06(2006.01)i

According to International Patent Classification (IPC) or to both national classification and IPC

**B.    FIELDS SEARCHED**

Minimum documentation searched (classification system followed by classification symbols)

G01N15/-

Documentation searched other than minimum documentation to the extent that such documents are included in the fields searched

Electronic data base consulted during the international search (name of data base and, where practicable, search terms used)

CNABS; CNTXT, DWPI, SIPOABS, CNKI: 校准, 污染度, 检测, 颗粒, 阈值, 计数, 基线, 电压, 电平, 油液, calibration, sensor, counting, fluid, patricle, stream, baseline, voltage

**C.    DOCUMENTS CONSIDERED TO BE RELEVANT**

| Category* | Citation of document, with indication, where appropriate, of the relevant passages | Relevant to claim No. |
|---|---|---|
| PX | CN 108169086 A (FATRI XIAMEN TECHNOLOGIES LTD.) 15 June 2018 (2018-06-15) claims 1-5 | 1-5 |
| X | CN 1682105 A (PARTICLE SIZING SYSTEMS, INC.) 12 October 2005 (2005-10-12) description, page 2, last paragraph to page 3, paragraph 2 | 1-5 |
| A | CN 101655457 A (MENG, GUOYING ET AL.) 24 February 2010 (2010-02-24) entire document | 1-5 |
| A | CN 101762447 A (DENG, KE) 30 June 2010 (2010-06-30) entire document | 1-5 |
| A | CN 105842142 A (SHENZHEN QINGHETAO TECHNOLOGY CO., LTD.) 10 August 2016 (2016-08-10) entire document | 1-5 |
| A | WO 2014005673 A1 (HYDAC FILTER SYSTEMS GMBH) 09 January 2014 (2014-01-09) entire document | 1-5 |

☐ Further documents are listed in the continuation of Box C.        ☑ See patent family annex.

| | | |
|---|---|---|
| *      Special categories of cited documents: | "T" | later document published after the international filing date or priority date and not in conflict with the application but cited to understand the principle or theory underlying the invention |
| "A"   document defining the general state of the art which is not considered to be of particular relevance | | |
| "E"   earlier application or patent but published on or after the international filing date | "X" | document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O"   document referring to an oral disclosure, use, exhibition or other means | | |
| "P"   document published prior to the international filing date but later than the priority date claimed | "&" | document member of the same patent family |

| Date of the actual completion of the international search | Date of mailing of the international search report |
|---|---|
| **15 January 2019** | **27 February 2019** |

| Name and mailing address of the ISA/CN | Authorized officer |
|---|---|
| **State Intellectual Property Office of the P. R. China** <br> **No. 6, Xitucheng Road, Jimenqiao Haidian District, Beijing** <br> **100088** <br> **China** | |
| Facsimile No. **(86-10)62019451** | Telephone No. |

Form PCT/ISA/210 (second sheet) (January 2015)

## INTERNATIONAL SEARCH REPORT
### Information on patent family members

| International application No. |
|---|
| **PCT/CN2018/118699** |

| Patent document cited in search report | | | Publication date (day/month/year) | Patent family member(s) | | | Publication date (day/month/year) |
|---|---|---|---|---|---|---|---|
| CN | 108169086 | A | 15 June 2018 | None | | | |
| CN | 1682105 | A | 12 October 2005 | US | 2004011975 | A1 | 22 January 2004 |
| | | | | EP | 1535040 | B1 | 17 August 2016 |
| | | | | US | 6794671 | B2 | 21 September 2004 |
| | | | | CN | 1682105 | B | 26 February 2014 |
| | | | | US | 2007010974 | A1 | 11 January 2007 |
| | | | | JP | 2010164572 | A | 29 July 2010 |
| | | | | JP | 4808963 | B2 | 02 November 2011 |
| | | | | JP | 2005533262 | A | 04 November 2005 |
| | | | | WO | 2004010113 | A1 | 29 January 2004 |
| | | | | EP | 1535040 | A1 | 01 June 2005 |
| | | | | EP | 2388569 | A1 | 23 November 2011 |
| | | | | US | 2005021244 | A1 | 27 January 2005 |
| | | | | JP | 4950314 | B2 | 13 June 2012 |
| | | | | CN | 103792168 | A | 14 May 2014 |
| | | | | CN | 103792168 | B | 01 June 2016 |
| | | | | US | 7127356 | B2 | 24 October 2006 |
| | | | | US | 7496463 | B2 | 24 February 2009 |
| | | | | AU | 2003239583 | A1 | 09 February 2004 |
| CN | 101655457 | A | 24 February 2010 | CN | 101655457 | B | 12 January 2011 |
| CN | 101762447 | A | 30 June 2010 | None | | | |
| CN | 105842142 | A | 10 August 2016 | None | | | |
| WO | 2014005673 | A1 | 09 January 2014 | US | 2015121994 | A1 | 07 May 2015 |
| | | | | US | 9518913 | B2 | 13 December 2016 |
| | | | | EP | 2870457 | A1 | 13 May 2015 |
| | | | | DE | 102012013255 | A1 | 08 May 2014 |

Form PCT/ISA/210 (patent family annex) (January 2015)